(19) Europäisches Patentamt
European Patent Office
Office européen des brevets

(11) **EP 4 675 494 A1**

(12) **EUROPEAN PATENT APPLICATION**

(43) Date of publication:
**07.01.2026 Bulletin 2026/02**

(21) Application number: **25179228.9**

(22) Date of filing: **27.05.2025**

(51) International Patent Classification (IPC):
**G06N 3/042** (2023.01)  **G06N 3/091** (2023.01)

(52) Cooperative Patent Classification (CPC):
**G06N 3/042; G06N 3/091; G16C 20/30;**
**G16C 20/70; G16C 60/00;** G06N 3/045

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **27.06.2024 JP 2024103749**

(71) Applicant: **FUJITSU LIMITED**
**Kawasaki-shi, Kanagawa 211-8588 (JP)**

(72) Inventor: **SAKAI, Yasufumi**
**Kawasaki-shi, 211-8588 (JP)**

(74) Representative: **Haseltine Lake Kempner LLP**
**Cheapside House**
**138 Cheapside**
**London EC2V 6BJ (GB)**

(54) **ACTIVE LEARNING PROGRAM, METHOD FOR ACTIVE LEARNING, AND INFORMATION PROCESSING APPARATUS**

(57) An active learning program causes a computer to execute a process including: extracting a first feature related to a structure of each of a plurality of materials by inputting a plurality of structure data associated one with each of the plurality of materials into an active learning neural network; obtaining a second feature related to energy of the structure of each of the plurality of the materials, using the active learning neural network, the second feature being based on the first feature; and determining, based on the first feature and the second feature of each of the plurality of materials, one or more structure data to be training data (2) for training an energy prediction neural network for predicting energy of a material from among the plurality of structure data.

FIG.10

## Description

TECHNICAL FIELD

The embodiment discussed herein relates to an active learning program, a method for active learning, and an information processing apparatus.

BACKGROUND ART

For material search, a technique of Materials Informatics (MI) is used. The reactivity of a given material is determined by its electronic structure. Nowadays, an electronic structure is calculated using density functional theory (DFT) simulation based on quantum mechanics, which demands a lot of computational complexity. In DFT simulations total electronic energy (sometimes simply referred to as "energy") of the material is an important object to be predicted. Alternatively, the adsorption energy, for example, is sometimes an object of prediction.

In recent years, a method has been proposed for rapidly obtaining an energy prediction value of a material by using a neural network, such as a graph neural network (GNN). However, supervised learning of a GNN model needs labeled training data. To calculate labels, a large-scale calculation (computation) of DFT simulation is performed. Accordingly, it is difficult to prepare an adequate amount of labeled training data from the viewpoints of computation workload and computation cost.

In machine learning, a technique called active learning is used to improve accuracy in energy prediction with less labeled training data. In active learning, a neural network (learning model) samples effective training data to improve its prediction performance. Therefore, active training improves prediction performance of the energy prediction value while reducing the amount of training data. Since the amount of the training data to be labeled can be reduced, it is possible to reduce the computation workload in DFT simulation which calculate the labels.

For example, an active learning that achieves efficient creation of structure data in an Au-Li binary system has been proposed (see Non-Patent Document 1).

One of preferable material searches discovers promising materials from a vast array of material data, each with diverse structure and diverse energies. For this purpose, a GNN for material search is trained with diverse training data.

CITATION LIST

NON-PATENT DOCUMENT

[Non-Patent Document 1] K. Shimizu, et al., "Phase stability of Au-Li binary systems studied using neural network potential", Phys. Rev. B,103, 094112(2021)

SUMMARY OF THE INVENTION

PROBLEMS TO BE SOLVED BY THE INVENTION

In the conventional active learning, training dataset which considers the diversity of the material structures is sampled by applying an active learning using geometric distances. However, in such conventional active learning, it is difficult to sample, as training data, data that is similar in structure but different in energy. This causes a difficulty in, if training data contains a small number of pieces, enhancing the accuracy of energy prediction values in a neural network.

With the above aspect in view, one of the objects of the embodiments is to determine training data that can reduce the number of data and enhance the accuracy of energy precision values by using a neural network.

MEANS TO SOLVE THE PROBLEM

According to an aspect of the embodiments, an active learning program causes a computer to execute a process including: extracting a first feature related to a structure of each of a plurality of materials by inputting a plurality of structure data associated one with each of the plurality of materials into an active learning neural network; obtaining a second feature related to energy of the structure of each of the plurality of the materials, using the active learning neural network, the second feature being based on the first feature; and determining, based on the first feature and the second feature of each of the plurality of materials, one or more structure data to be training data for training an energy prediction neural network for predicting energy of a material from among the plurality of structure data.

EFFECT OF THE INVENTION

In one aspect, the present embodiments can determine training data that can reduce the number of data pieces and can enhance the accuracy of energy precision values by using a neural network.

BRIEF DESCRIPTION OF DRAWINGS

FIG. 1 is a diagram illustrating an overview of supervised learning of an energy prediction NN;
FIG. 2 is a diagram illustrating an overview of a method of training a NN applied with an active learning;
FIG. 3 is a diagram illustrating a method for determining training data in an active learning in a comparative example;
FIG. 4 is a diagram illustrating an example in which structure data forms a uniform population;
FIG. 5 is a diagram illustrating an example in which structure data forms a population consisting of multiple cluster population;

FIG. 6 is a diagram illustrating an example of a relationship between structural features and energy;

FIG. 7 is a diagram illustrating an example a feature space of a data set OC22;

FIG. 8 is a diagram illustrating an example of a mean absolute error of energy prediction values of various data regions in the comparative example;

FIG. 9 is a block diagram schematically illustrating an example of a hardware (HW) configuration of a computer of a first embodiment;

FIG. 10 is a diagram illustrating an example of an active learning according to the first embodiment;

FIG. 11 is a block diagram schematically illustrating an example of a functional configuration of the computer of the first embodiment;

FIG. 12 is a flow chart illustrating an example of a process of the active learning implemented by the computer of the first embodiment;

FIG. 13 is a diagram illustrating a result of an experiment on a mean absolute error of energy prediction values of the active learning in the first embodiment;

FIG. 14 is a block diagram schematically illustrating an example of a functional configuration of a computer of a second embodiment;

FIG. 15 is a flow chart illustrating an example of a process of an active learning implemented by the computer according to the second embodiment; and

FIG. 16 is a diagram illustrating a result illustrating an effect in reducing a mean absolute error of energy prediction values of the active learning in the second embodiment.

DESCRIPTION OF EMBODIMENT(S)

Hereinafter, the embodiments of the present disclosure will be described, referring to the accompanying drawings. However, the embodiments described below are merely illustrative and are not intended to exclude the application of various modifications and techniques not explicitly described below. For example, the present embodiment can be variously modified and implemented without departing from the scope thereof. In the drawings to be used in the following description, like reference numbers designate the same or substantially same parts and elements, unless otherwise specified.

First Embodiment

Description of Neural Network (NN) for Energy Prediction and Active Learning

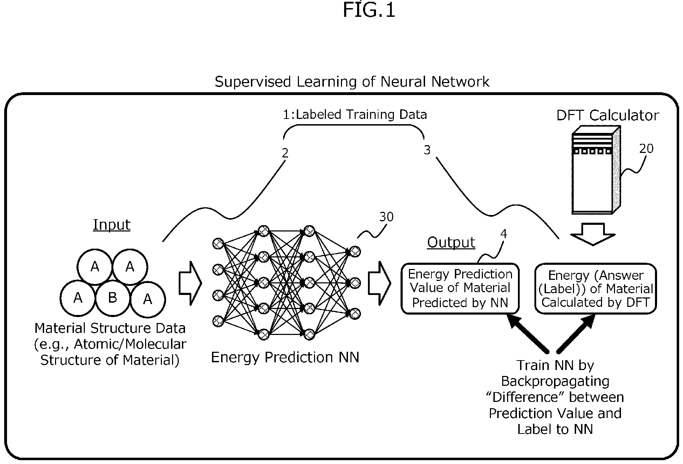

FIG. 1 is a diagram illustrating an overview of supervised learning of an energy prediction NN. Energy values of the materials of multiple structure data 2 corresponding one to each of the multiple materials are calculated using density functional theory (DFT) simulation based on quantum mechanics by a DFT calculator 20. The calculated energy values are labels 3 (answers) of the structure data 2. The structure data 2 is an example of training data. The combination of the structure data 2 and the labels 3 serves as labeled training data 1. The structure data 2 of the materials is data about atomic or molecular structures of the materials.

The structure data 2 is inputted as training data into an energy prediction NN 30. The energy prediction NN 30 outputs energy prediction values 4 of the materials. The energy prediction NN 30 is trained by back propagating an "error" between the label 3 that are obtained by DFT computation and the energy prediction value 4 through the NN. The energy prediction NN 30 may be a machine learning model that is trained by machine learning (ML).

The energy prediction NN 30 may be, for example, a Graph Neural Network (GNN). In one example, a Polarizable atom interaction Neural Network (PaiNN) and an EquiformerV2 are used as the energy prediction NN 30. EquiformerV2 is one type of NN that regards a symmetry of data as a powerful inductive bias and incorporates the symmetry into a network design.

The technique of PaiNN is detailed in, for example, "K. T. Schuett, O. T. Unke, and M. Gastegger, "Equivariant message passing for the prediction of tensorial properties and molecular spectra," in Proceedings of the International Conference on Machine Learning, pp. 9377-9388, 2021", and the technique of EquiformerV2 is detailed in, for example, "Y.-L. Liao, B. Wood, A. Das, and T. Smidt, "EquiformerV2: Improved equivariant transformer for scaling to higher-degree representations," arXiv preprint arXiv:2306.12059, 2023". So, detailed description thereof is omitted here.

In response to the input of the structure data of the materials thereto, the energy prediction NN 30 extracts the feature of the structure of each material. The dimension of the feature (hereinafter referred to as a structural feature) of the structure to be extracted is an N dimension (where N is the number of atoms of each inputted material structure). The dimension of a feature is reduced to, for example, two dimensions by means of densMAP, which is a method of embedding that reflects density of a distribution of high-dimensional data. The energy prediction NN 30 outputs the energy prediction value of the structure of each material on the basis of the structural feature. The energy prediction NN 30 is not limited to PaiNN and EquiformerV2.

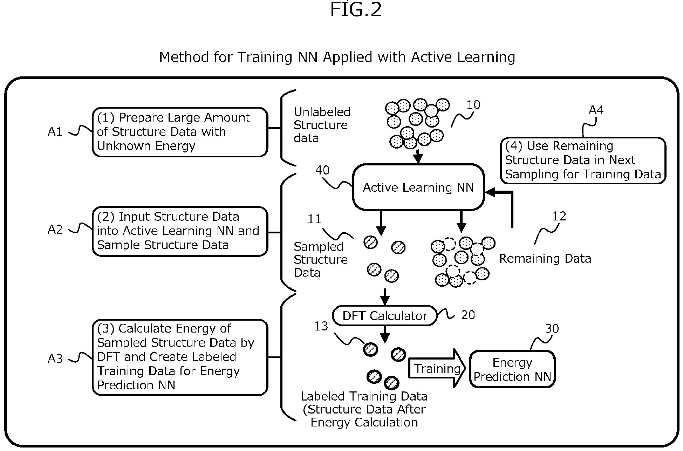

FIG. 2 is a diagram illustrating an overview of a method of training the NN applied with an active learning. In machine learning, an active learning is applied to enhance an accuracy in the energy prediction using the labeled training data 1 with reduced data number.

A large amount of structure data 10 with unknown energy are prepared (A1). Here, the structure data is multiple structure data corresponding one to each of multiple materials. The multiple structure data 10 are inputted into an active learning NN 40 (A2). The active learning NN 40 samples (selects) one or more structure data 11 from among the multiple structure data 10 (N2). In other words, the active learning NN 40 determines the sampled structure data as training data for training the energy prediction NN 30.

The active learning NN 40 is a NN for active learning and may be also a machine learning model. The active learning NN has the same configuration as the energy prediction NN 30. As the active learning NN 40, the energy prediction NN 30 or another energy prediction NN may be used.

The DFT calculator 20 calculates an energy value of each material in structure data as the label 3 of the training data by executing DFT simulation computation based on the structure data 11 determined to be the training data (A3). In other words, labeled training data 13 is created. Here, remaining structure data 12 left not being sampled in A2 may be used for the next sampling of training data (A4).

Consequently, although the computational complexity of DFT simulation computation is reduced by reducing the number of training data, the accuracy of the energy prediction value can be enhanced.

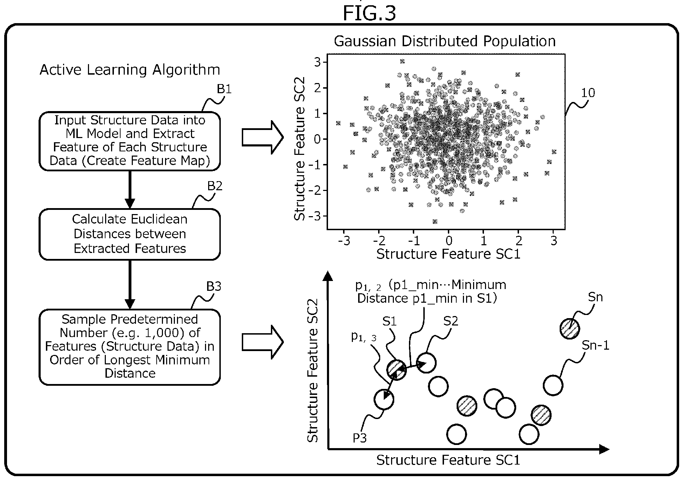

FIG. 3 is a diagram illustrating a method for determining training data in an active learning in a comparative example. By inputting each of the multiple structure data 10 into the active learning NN 40 which is a ML model, the feature for each structure data 10 is extracted (B1).

The feature of each structure data 10 may be reduced in dimension by densMAP and consequently have two dimensions, for example, SC1 and SC2.

In a feature space, the Euclidean distances between the extracted features are calculated (B2). As illustrated in FIG. 3, respective distances $p_{1,2}$, $p_{1,3}$,..., $p_{1,n-1}$, and $p_{1,n}$ between a point S1 indicating a structural feature of interest and respective points S2-Sn (n is the number of structure data 10) indicating the multiple structural features other than S1 in the feature space calculated.

Among the distances $p_{1,2}$, $p_{1,3}$,..., $p_{1,n-1}$, and $p_{1,n}$, the minimum distance ($p_{1,2}$ in the example of FIG. 3) is calculated. Similarly, the respective minimum distances of S2-Sn are calculated. One or more structural features are sampled in descending order of minimum length (i.e., in the order of longer minimum distance) (B3). The number of structural features to be sampled may be determined in advance. For example, 1000 structural features are selected. One or more structure data corresponding to the sampled structural features are determined as training data.

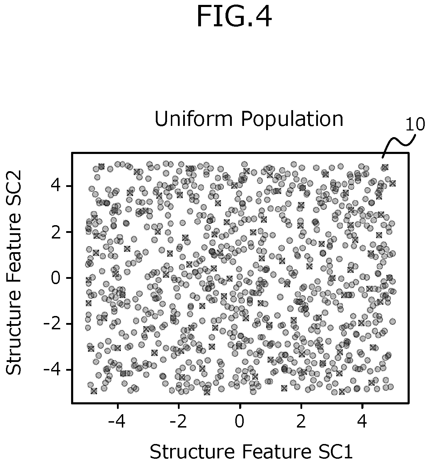

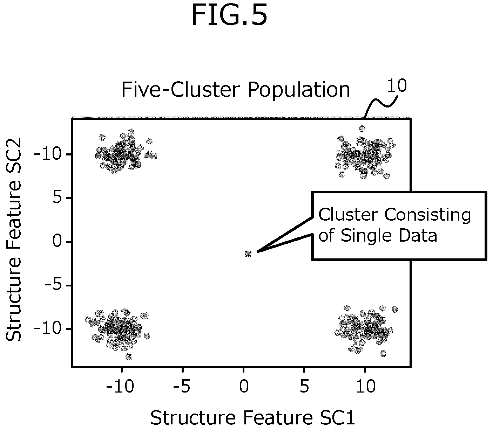

The method of determining the training data illustrated in FIG. 3 can capture the diversity of structure of the multiple structure data 10 regardless of the populations of the multiple structure data illustrated in FIG. 4 and FIG. 5 as well as in a situation where the structured data forms the Gaussian distribution population as illustrated in FIG. 3. FIG. 4 is a diagram illustrating an example in which structure data forms a uniform population. FIG. 5 is a diagram illustrating an example in which structure data forms a population consisting of multiple cluster population. In FIG. 5, the multiple structure data 10 forms four clusters each consisting of multiple points and a center cluster consisting of single point, which means five clusters in total. The method for determining of FIG. 3 samples training data from the five clusters and can thereby determine training data free from deviation.

The method for determining training data of FIG. 3 can determine the training data, considering the diversity of the structures. However, the method for determining of FIG. 3, which samples data by utilizing mainly the features of the structure of materials, has difficulty in considering the diversity of energies that the materials have.

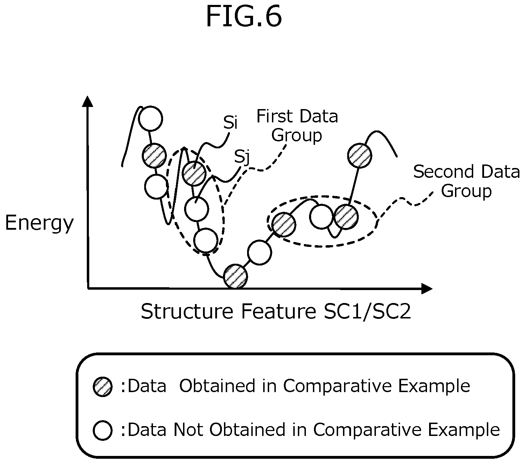

FIG. 6 is a diagram illustrating an example of a relationship between structural features (SC1, SC2) and energy. As in a first data group, materials may have similar structural feature but different energies. On the other hand, as in a second data group, materials may have different structural feature but similar energies. In FIG. 6, hatched structural features are sampled as the training data when the comparative method of FIG. 3 is applied, and white-circular structural features are not sampled as the training data when the comparative method of FIG. 3 is applied. For example, if the point Si is sampled as the training data, the point Sj having a structural feature close to that of the point Si is less likely sampled as the training data. The structure data of this point Sj differs significantly from data and energy corresponding to the point Si collected by the method for determining of FIG. 3. Consequently, the accuracy in precision on the structure data of Sj is lowered.

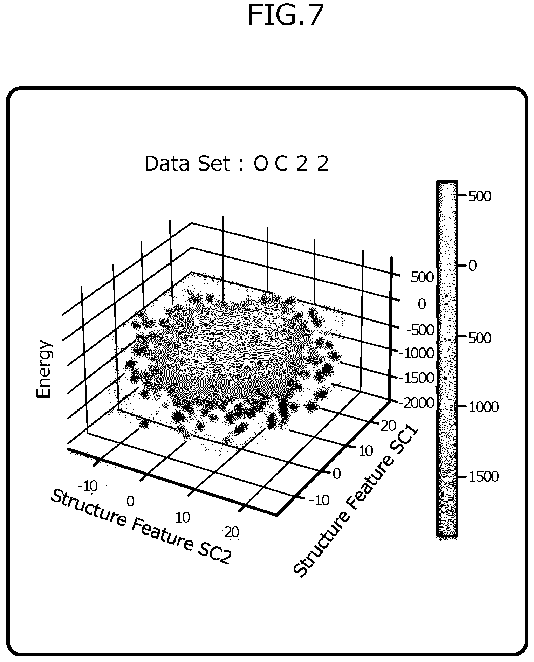

FIG. 7 is a diagram illustrating an example a feature space of a dataset OC22. The dataset OC22 (Open Catalyst 2022) is one of the largest catalyst datasets for predicting the total energy of catalysts opened to public.

As illustrated in FIG. 7, the structure data in the OC22 may have similar structural features (SC1 and SC2), but may have different energies corresponding to the vertical axis. Then, if the energy prediction NN 30 is trained with training data determined from a region in which the structural features are similar but the energies are different in the method for determining of FIG. 3, the accuracy in the energy prediction by the energy prediction NN 30 may be lowered.

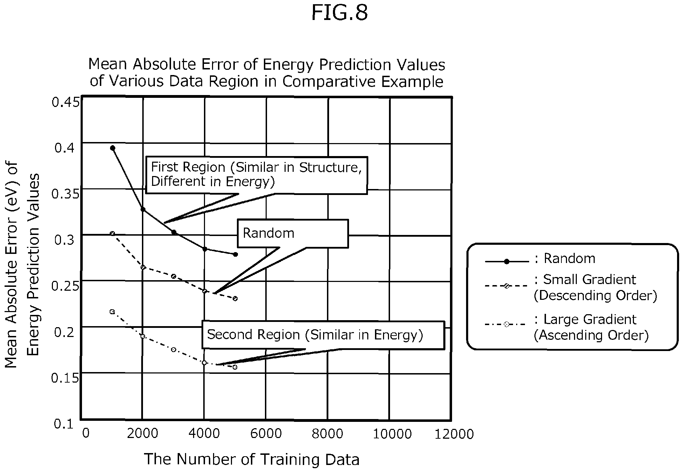

FIG. 8 is a diagram illustrating an example of a mean absolute error of energy prediction values of various data regions in the comparative example. An abscissa represents the number of training data, and an ordinate represents the Mean Absolute Error (MAE) of the energy prediction values predicted by a trained energy prediction NN 30. The mean absolute error of the energy prediction values is also referred to as a prediction error. As the number of training data increases, the MAR of the energy prediction tend to decrease.

As illustrated in FIG. 8, a first data group that is similar in structure but differs in energy has a larger MAE than a second data group that is similar in energy and a data group consisting of data randomly sampled, so that the accuracy in energy prediction value may be lowered. The present first embodiment can enhance the accuracy of the energy prediction value with the neural network while reducing the number of training data by making it possible to sample, as the training data, data similar in structure but largely differing in energy.

Example of Hardware Configuration:
The function of a computer 100 of the first embodiment may be achieved by one computer or by two or more computers. Further, at least a part of the functions of the computer 100 may be implemented using Hardware (HW) resources and Network (NW) resources provided by cloud environment.

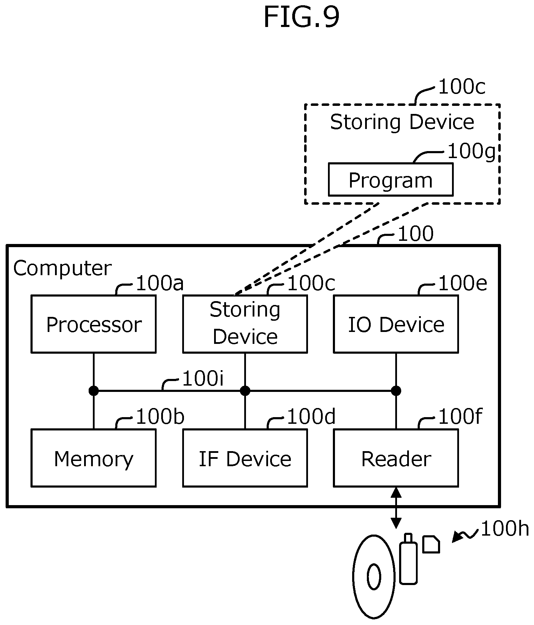

FIG. 9 is a block diagram schematically illustrating an example of a hardware (HW) configuration of the computer 100 that achieves the function of the computer 100 of the first embodiment. If multiple computers are used as the HW resources for achieving the functions of the computer 100, each of the computers may include the HW configuration illustrated in FIG. 9.

As illustrated in FIG. 9, the computer 100 may illustratively include, as the HW configuration, a processor 100a, a memory 100b, a storing device 100c, an Interface (IF) device 100d, an Input/Output (IO) device 100e, and a reader 100f.

The processor 100a is an example of an arithmetic processing device that performs various types of control and calculations. The processor 100a may be mutually communicably connected to each of the blocks in the computer 100 via a system bus 100i. The processor 100a may be a multi-processor including multiple processors or a multi-core processor including multiple processor cores, or may have a structure including two or more multi-core processors.

The processor 100a may be any one of integrated circuits (ICs) such as Central Processing Units (CPUs), Micro Processing Units (MPUs), Graphics Processing Units (GPUs), Accelerated Processing Units (APUs), Digital Signal Processors (DSPs), Application Specific Integrated Circuits (ASICs), and Field Programmable Gate Arrays (FPGAs), or combinations of two or more of these ICs. Here, the GPUs may be General Purpose computing on Graphics Processing Units (GPGPUs).

The memory 100b is an example of a hardware device that stores various pieces of data and information of a program. An example of the memory 100b is one of a volatile memory such as a Dynamic Random Access Memory (DRAM) and a non-volatile memory such as a persistent Memory (PM) or the both.

The storing device 100c is an example of a hardware device that stores information such as various data, programs, and the likes. Examples of the storing device 100c may be various storing devices including a magnetic disk device such as a Hard Disk Drive (HDD), a semiconductor drive device such as a Solid State Drive (SSD), a nonvolatile memory, and the like. The nonvolatile memory may be, for example, a flash memory, a Storage Class Memory (SCM), a Read Only Memory (ROM), and the like.

The storing device 100c may store a program 100g (active learning program) that implements all or a part of various functions of the computer 100. The program 100g may include, for example, an Operating System (OS) in addition to the active learning program.

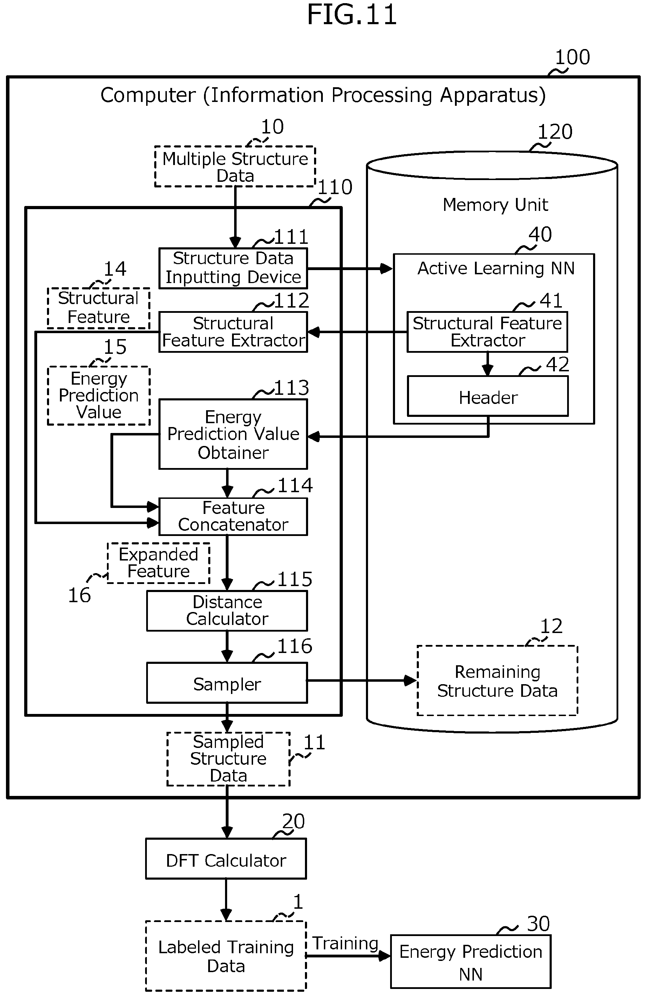

For example, the processor 100a may achieve the function of a controller (controller 110 of FIG. 11 to be detailed below) of the computer 100 by expanding the program 100g stored in the storing device 100c on the memory 100b and executing the expanded program 100g.

The computer 100 may execute each process of the active learning by executing the active learning program.

The IF device 100d is an example of a communication IF that controls connections and communications between the computer 100 and other devices.

For example, the IF device 100d may include an applying adapter conforming to Local Area Network (LAN) such as Ethernet® or optical communication such as Fibre Channel (FC). The applying adapter may be compatible with either or both of wireless and wired communication schemes.

Furthermore, the program 100g may be downloaded from a network to the computer 100 through the communication IF device 100d and be stored in the storing device 100c.

The IO device 100e may include one or both of an input device and an output device. Examples of the input device include a keyboard, a mouse, and a touch panel. Examples of the output device include a monitor, a projector, and a printer. The IO device 100e may include, for example, a touch panel that integrates an input device and an output device with each other.

The reader 100f is an example of a reader that reads information of data and programs recorded on a recording medium 100h. The reader 100f may include a connecting terminal or device to which the recording medium 100h may be connected or inserted. Examples of the reader 100f include an applying adapter conforming to, for example, a Universal Serial Bus (USB), a drive apparatus that accesses a recording disk, and a card reader that accesses a flash memory such as an SD card. The program 100g may be stored in the recording medium 100h. The reader 100f may read the program 100g from the recording medium 100h and store the read program 100g into the storing device 100c.

Examples of the recording medium 100h illustratively include a non-transitory computer-readable recording medium such as a magnetic/optical disk, and a flash memory. Examples of the magnetic/optical disk include a flexible disk, a Compact Disc (CD), a Digital Versatile Disc (DVD), a Blu-ray disk, and a Holographic Versatile Disc (HVD). Examples of the flash memory include a semiconductor memory such as a USB memory and an SD card.

The HW configuration of the computer 100 described above is exemplary. Accordingly, the computer 100 may appropriately undergo increase or decrease of the HW devices (e.g., addition or deletion of arbitrary blocks), division, integration in an arbitrary combination, or addition or deletion of the bus.

Overview of Active Learning Process of First Embodiment

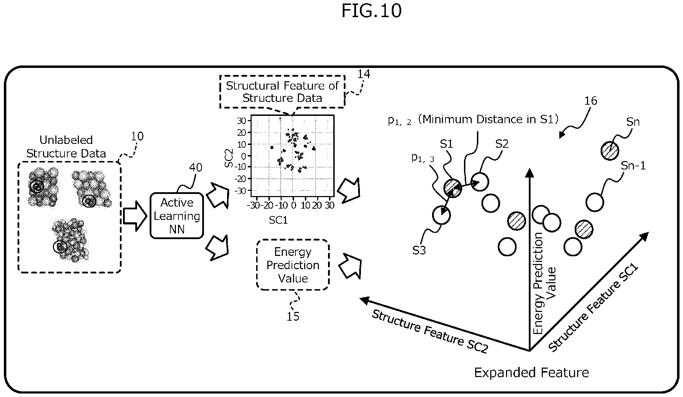

FIG. 10 is a diagram illustrating an example of an active training according to the first embodiment of the present disclosure. Also in the first example, similarly to the comparative example, multiple structure data 10 corresponding one to each of multiple materials are inputted into the active learning NN 40. The structure data 10 is data about the atomic or molecular structures of the materials.

The active learning NN 40 is exemplified by a GNN. The active learning NN 40 has the same configuration as the energy prediction NN 30 using PaiNN and EquiformerV2, for example. A structural feature 14 is generated for each of structure data by the active learning NN 40. The structural feature 14 is an example of a first feature related to the structure of each material.

In the active learning NN 40, the structural feature 14 is extracted from the structure data 10. The active learning NN 40 calculates an energy prediction value 15 based on the structural feature 14. The energy prediction value 15 is an example of a second feature related to energy of the structure of each material, which is calculated on the basis of the first feature with the active learning NN.

A controller 110 (see FIG. 11) determines, based on the first feature and the second feature of each material, one or more structure data from among multiple structure data to be training data for training the energy prediction NN that predicts the energy of the material.

In order to obtain data that captures the structure and energy diversity, the prediction values 15 as well as the structural features 14 are added as features to be used to calculate the distances. For example, if the structural feature 14 is two-dimensional, a three-dimensional expanded feature 16 is created by concatenating the structural feature 14 with the energy prediction value 15. The expanded feature 16 is an example of a concatenated feature generated by concatenating the first feature of each material with the second feature of the material.

In a feature space expanded to include the energy prediction values as a dimension, the Euclidean distances between the respective expanded features 16 are calculated. As illustrated in FIG. 10, in the extended feature space, respective distances $p_{1,2}$, $p_{1,3}$, $\cdots p_{1,n-1}$,

$p_{1,n}$ between a point S1 indicating an expanded feature of interest and respective points S2-Sn (n is the number of structure data 10) indicating the multiple structural features other than S1 in the expanded feature space calculated.

Among the distances $p_{1,2}$, $p_{1,3}$,..., $p_{1,n-1}$, $p_{1,n}$, the minimum distance ($p_{1,2}$ in FIG. 10) is calculated. Similarly, the respective minimum distances of S2-Sn are calculated. One or more expanded structural features are sampled in descending order of the minimum distance (i.e., in order of longer minimum distance). The number of expanded features to be sampled may be determined in advance. One or more structure data corresponding to the sampled expanded feature are determined as the training data.

Example of Functional Configuration of First Embodiment

FIG. 11 is a block diagram schematically illustrating an example of a functional configuration of the computer 100 of the first embodiment. The computer 100 is an example of an information processing apparatus.

As illustrated in FIG. 11, the computer 100 includes a controller 110 and a memory unit 120. The controller 110 includes a structure data inputting device 111, a structural feature extractor 112, an energy prediction value obtainer 113, a feature concatenator 114, a distance calculator 115, and a sampler 116. The DFT calculator 20 may be provided as one function of the computer 100.

The memory unit 120 is an example of a storing region and stores various data that the controller 110 uses. The memory unit 120 may be implemented by, for example, one or more storing region included in one or both of the memory 100b and the storing device 100c illustrated in FIG. 9.

As illustrated in FIG. 11, the memory unit 120 may be illustratively provided with the active learning NN 40. The memory unit 120 may store, for example, the structure data 12 that remains unsampled in the proceeding of the active learning.

The structure data inputting device 111 inputs multiple structure data 10 corresponding one to each of the multiple materials into the active learning NN 40.

The active learning NN 40 may be a GNN. In this case, each node (corresponding to an atom) shares information with other nodes by repeating message passing that receives information from one or more peripheral nodes (corresponding to atoms) and updates its own information. As a result, the nodes can handle the characteristics of the entire graph. The GNN may include, as intermediate layers, a convolutional layer and a pooling layer. In the convolutional layer, node characteristics are updated on the basis of the message passing. On the other hand, the pooling layer aggregates the node characteristics and extracts the structural features 14 of the overall materials, which is the characteristic of the entire graph. The specific active learning NN 40 is the same as the energy prediction GNN, such as PaiNN and EquiformerV2, and the detailed description thereof is omitted here.

The intermediate layers of the active learning NN 40 function as a structural feature extractor 41 that extracts the structural feature 14 from the structure data 10. The active learning NN 40 has a header 42. The header 42 calculates and outputs the energy prediction value 15 corresponding to the material structure on the basis of the structural feature 14 created in the intermediate layers.

The structural feature extractor 112 extracts the structural feature 14 from the structural feature extractor 41 of the active learning NN 40, that is, the intermediate layer such as the pooling layer.

The energy prediction value obtainer 113 calculates the energy prediction value, which is the output from the header 42 of the active learning NN 40.

The feature concatenator 114 normalizes the structural feature 14 and the energy prediction value 15, and then concatenates the normalized feature and value to thereby create a expanded feature 16. The term "concatenating" may mean creation of a new feature vector containing the basis (m basis in this example) of the feature vector of the structural feature 14 and the basis (one basis in this example) of the feature vector of the energy prediction value 15 (energy feature).

The distance calculator 115 calculates the Euclidean distances between the expanded features 16 in the feature space extended to include the energy prediction value 15 as one of the dimensions. As illustrated in FIG. 10, the distance calculator 115 calculates, for each of the concatenated features, the minimum distance among the distances between a point indicating the expanded feature 16 of interest and respective points one indicating each of multiple concatenated features in the extended feature space.

The sampler 116 samples one or more expanded features 16 in descending order of the minimum length (i.e., in order of having longer minimum length). The sampler 116 determines one or more structure data corresponding to the sampled expanded features to be the training data.

The DFT calculator 20 calculates a value of the energy of the material in the structure data 11 to be the label 3 of the training data by a density functional theory calculation performed on the structure data 11 sampled to be the training data by the sampler 116.

Operation in First Embodiment

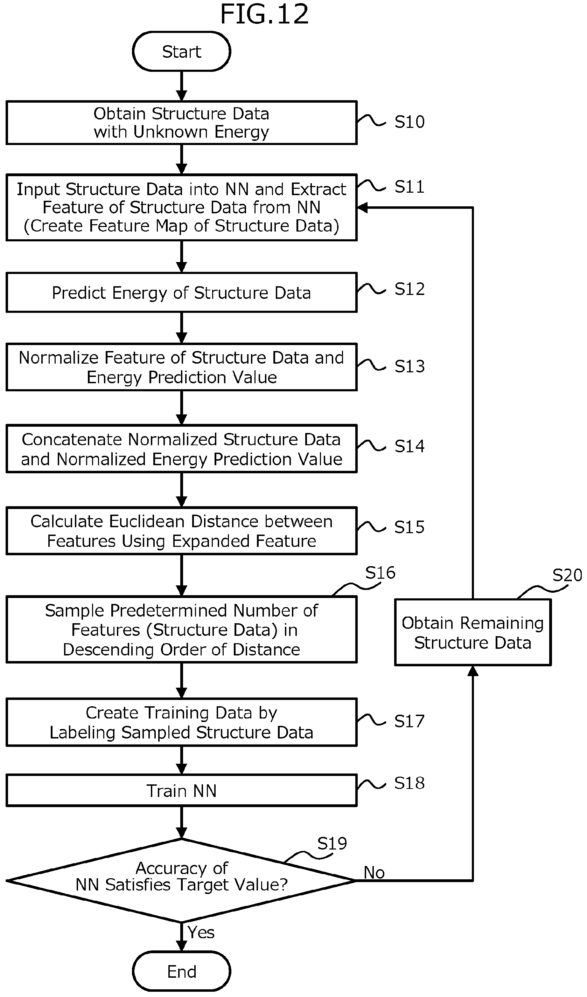

FIG. 12 is a flow chart illustrating an example of a process of the active learning implemented by the computer of the first embodiment.

The structure data inputting device 111 obtains structure data 10 with unknown energy (Step S10).

The structure data inputting device 111 inputs the obtained structure data 10 into the active learning NN 40, and the structural feature extractor 112 extracts the structural feature 14 of the structure data (Step S11). In other words, the structural feature extractor 112 creates a feature map about the structure of the structure data.

The energy prediction value obtainer 113 calculates (predicts) the energy prediction value 15, using the header 42 of the active learning NN 40 (Step S12).

The feature concatenator 114 normalizes the structural feature 14 and the energy prediction value 15 of the structure data (Step S13). This provides proper weighting for concatenation of the structural feature 14 and the energy prediction value 15. This means that the importance of the feature and the energy prediction value of the structure can be made matched.

The feature concatenator 114 concatenates the normalized structural feature 14 and the normalized energy prediction value 15 and thereby creates the expanded feature 16 (Step S14).

The distance calculator 115 calculates the Euclidean distances between the expanded features in the feature space in which the dimensions are extended. The distance calculator 115 calculates, for each of the concatenated features, the minimum distance among the distances between a point indicating the expanded feature 16 of interest and respective points one indicating each of multiple concatenated features in the extended feature space (Step S15).

The sampler 116 samples the predetermined number of expanded features 16 in descending order of the minimum length (Step S16). The sampler 116 determines the structure data 11 corresponding to the sampled expanded features to be the training data.

The DFT calculator 20 attaches the calculated label 3 to sampled structure data 11, which means labeling of the sampled structure data 11 (Step S17). Accordingly, the controller 110 creates the labeled training data 1. As illustrated in FIG.1, the controller 110 trains the energy prediction NN 30 using the labeled training data 1 (Step S18).

The controller 110 obtains accuracy data of the energy prediction value predicted by the energy prediction NN 30. The accuracy data may be exemplified by a MAE. If the accuracy of the energy prediction value predicted by the energy prediction NN 30 does not satisfy a target value (see No route of Step S19), the controller 110 obtains remaining structure data 12 (Step S20), and repeats the processes from Step S11 to Step S19. On the other hand, if the accuracy of the energy prediction value predicted by the energy prediction NN 30 satisfies the target value (see Yes route of Step S19), the controller 110 completes the process. The state where the accuracy does not satisfy the target value corresponds to the MAE of a predetermined value or more, and the state where the accuracy satisfies the target value corresponds to the MAE less than the predetermined value.

Effect of First Embodiment

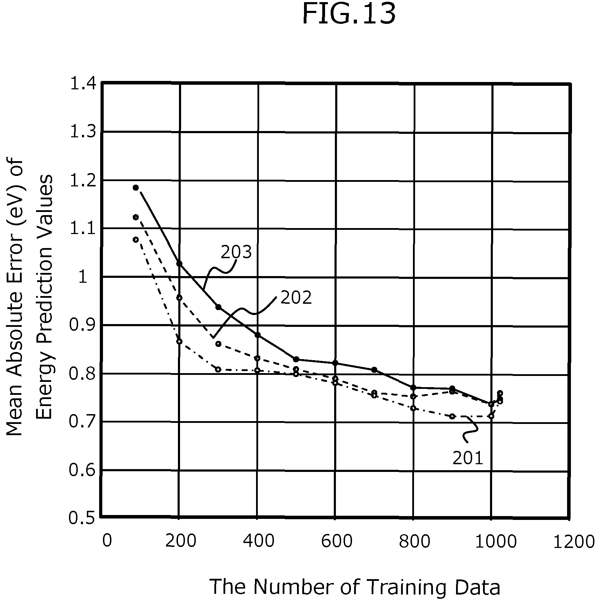

FIG. 13 is a diagram illustrating a result of an experiment on a mean absolute error of energy prediction values by means of the active learning in the first embodiment. FIG. 13 illustrates an example in which 100 (pieces of) training data are determined though the active learning and the energy of catalysts for generation of ammonia is predicted with the determined training data.

In FIG. 13, a polyline 201 represents a result of training using the training data determined in the active learning of the first embodiment, and a polyline 202 represents a result of training using the training data determined in the active learning of the comparative example illustrated in FIG. 3. A polyline 203 indicates the result when data is selected randomly.

By training using the training data determined in the active learning of the first embodiment, the mean absolute error in the energy prediction NN 30 can be reduced as compared with training using the training data determined in the active learning of the comparative example.

As a method other than the comparative example of FIG. 3 and the random sampling, a result of uncertainty sampling based on the variance of the predicted energy obtained by Gaussian process regression (GPR) was also compared with the result of the active learning method of the first example using the OC22 dataset. The method for active learning of the first embodiment, even when the amount of training data was limited, achieved a lower MAE than the uncertainty sampling.

A structural dataset such as a catalyst for nitrogen reduction reaction (NRR) and a catalyst for oxygen reduction reaction (ORR) other than OC22, is used to predict adsorption energy in a catalyst-adsorbate system. Also in such cases, the method of the first embodiment, which however has a limited amount of training data, achieved the lowest MAE among all the methods.

According to the active learning technique of the present embodiment, the processor 100a inputs each of multiple structure data 10 corresponding to one of the multiple materials into the active learning NN to extract the structural feature 14 (that is, the first feature) related to the structure of each material. The processor 100a calculates the energy prediction value 15 (i.e., the second feature) related to the energy in the structure of each material on the basis of the structural feature 14, using the active learning NN 40. The processor 100a determines, based on the structural feature 14 and the energy prediction value 15 of each material, one or more structure data 11 from among multiple structure data 10 to be the training data for training the energy prediction NN 30 that predicts the energy of the material.

This can consider the feature of the energy as well as the feature related to the structure of each material. Since the diversity of energy can be considered, structure data similar in structure but largely differing in energy can be determined to be the training data. Accordingly, by training with the determined training data, the prediction error of the energy prediction NN can be improved even if the amount of the training data is small.

In the process of determining the training data, the processor 100a generates the expanded feature 16 (concatenated feature) in which the structural feature 14 (first feature) corresponding each material is concatenated with the energy prediction value 15 (second feature) of the material. The processor 100a calculates, for each of the expanded features 16, the minimum distance among the distances of a point representing the expanded feature 16 from the respective points representing the multiple concatenated features in the extended feature space. The processor 100a samples one or more expanded feature in descending order of the minimum distance for each of the expanded features, and determines one or more structure data corresponding to the sampled expanded features to be the training data.

This can consider the feature of the energy as well as the feature related to the structure of each material on the basis of the distances in the same feature space, so that the scheme of the present embodiment can be easily implemented onto a computer and can reduce the processing load.

The second feature is the energy prediction value 15 of the material comprising the structure and is calculated by the active learning NN 40.

This makes it possible to use the energy prediction value 15 calculated by the active learning NN 40 and the structural feature 14 extracted in the active learning NN 40, so that the scheme of the present embodiment can be implemented to a computer with ease and can reduce the processing load.

Furthermore, the processor 100a calculates the value of energy of the material in the structure data 11 to be the label 3 of the training data by performing the density functional theory calculation based on the structure data 11 determined to be the training data.

As a result, this can enhance the accuracy in the training of the energy prediction NN 30 on the basis of the label 3 obtained by the density functional theory calculation and can properly narrow the number of training data, so that the processing load on the computer and the cost in the density functional theory calculation can be reduced.

Second Embodiment

Example of Functional Configuration in Second Embodiment

The process in which the controller 110 determines, based on the first feature and the second feature of each material, one or more structure data from among multiple structure data to be training data for training the energy prediction NN that predicts the energy of the material is not limited to the process of the first embodiment. In a second example, an energy gradient is used as the second feature.

The hardware configuration of the computer 100 in the second embodiment is the same as that of the first example illustrated in FIG. 9, so repetitious explanation thereof is omitted here.

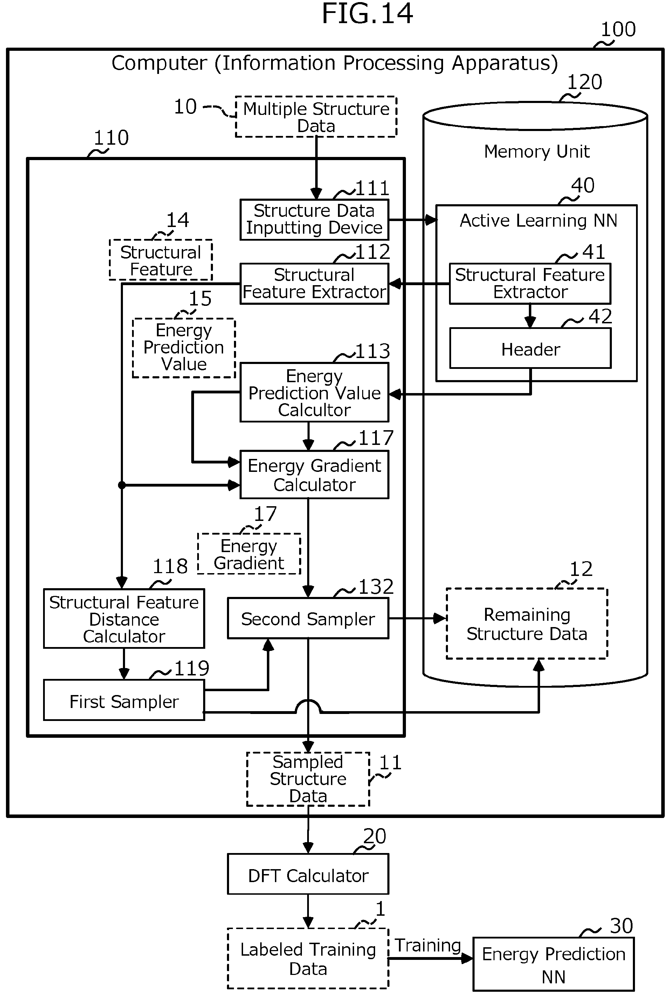

FIG. 14 is a block diagram schematically illustrating an example of a functional configuration of the computer 100 of the second embodiment. In the second embodiment, the feature concatenator 114, the distance calculator 115, and sampler 116 illustrated in FIG. 11 are replaced with an energy gradient calculator 117, a structural feature distance calculator 118, a first sampler 119, and a second sampler 132. The remaining configuration other than the above are the same as those of the first example.

The energy gradient calculator 117 calculates an energy gradient 17. The energy gradient 17 is the ratio of a change $\Delta e_{i,j}$ in the energy prediction value 15 of the material comprising the structure for each distance $\Delta p_{i,j}$ between the point $S_i$ representing the structural feature 14 and the point $S_j$ representing another structural feature 14 other than the point $S_i$ in the feature space.

The energy gradient calculator 117 calculates the energy gradient between the point $S_i$ and the point $S_j$ that represent the structural features 14 by the following expression.

$$\frac{\Delta e_{i,j}}{\Delta p_{i,j}} = \left| \frac{e_i - e_j}{\sqrt{\sum_{n=1}^{D}\left(p_{i,n} - p_{j,n}\right)^2}} \right|$$

The energy gradient calculator 117 calculates the energy gradients 17 of a point Si representing the structural feature 14 of interest with respect to the points S1, S2,..., Si-1, Si+1,..., Sn (where n is the number of structure data 10) representing multiple structural features 14 other than Si in the feature space. When i=1, i.e., for Si, the respective energy gradients 17 are calculated by using the distances $p_{1,2}$, $p_{1,3}$, ..., $p_{1,n-1}$, and $p_{1,n}$ as denominators and the energy changes $e_{1,2}$, $e_{1,3}$, ..., $e_{1,n-1}$, and $e_{1,n}$ as the respective corresponding numerators. At the point S1, the maximum energy gradient 17 is calculated. Similarly, the maximum energy gradient 17 is calculated for each of S2-Sn. One or more structural features 14 are sampled in descending order of maximum energy gradient 17 (i.e., in order of larger energy gradient 17). The number of structural features to be sampled may be determined in advance.

The structural feature distance calculator 118 calculates the Euclidean distances between the extracted structural features 14 in the feature space. Likewise the case of FIG. 3, the structural feature distance calculator 118 calculates the distances $p_{1,2}$, $p_{1,3}$, $\cdots p_{1,1-n}$, $p_{1,n}$ of a point S1 representing a structural feature of interest from the respective points S2-Sn representing multiple structural features other than S1 in feature space.

The structural feature distance calculator 118 calculates the minimum distance among the distances $p_{1,2}$, $p_{1,3}$,..., $p_{1,1-n}$, $p_{1,n}$. Similarly, the minimum distance is calculated for each of S2-Sn. The first sampler 119 samples one or more structural features in descending order of minimum length (i.e., in order of having longer minimum distance). The number of structural features to be sampled may be predetermined.

The second sampler 132 may sample one or more structural features 14 for the structure data sampled by the first sampler 119 in descending order of the maximum energy gradient 17. The first sampler 119 may select the candidates based on the Euclidean distances, and the second sampler 132 may sample structure data in descending order of energy gradient from the candidates.

According to the active learning of the second embodiment, after the data is narrowed by using "Euclidean distances" without depending on the population distribution, the second sampler 132 samples training data to be finally used by the energy gradient calculator 117.

Operation of Second Embodiment

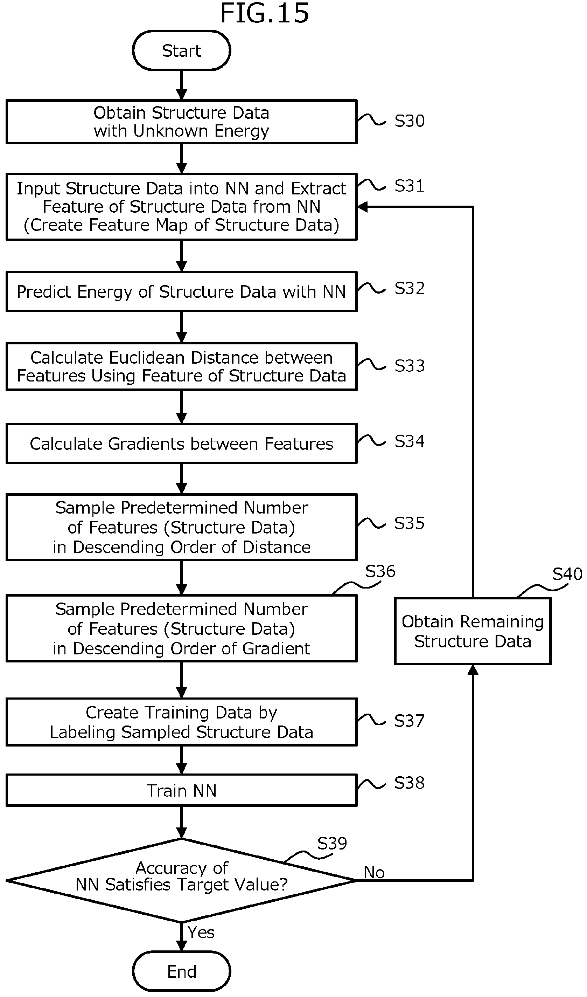

FIG. 15 is a flow chart illustrating an example of a process of an active learning implemented by the computer according to the second embodiment.

Steps S30-S32 in FIG. 15 are the same as the Steps S10-S12 in FIG. 12, respectively.

The structural feature distance calculator 118 calculates the Euclidean distances between the extracted structural features 14 in the feature space (Step S33).

The energy gradient calculator 117 calculates the energy gradients 17 between the structural features 14 (Step S34).

The first sampler 119 samples one or more structural features in order of having longer minimum distance (Step S35). The number of structural features to be sampled may be determined in advance.

The second sampler 132 samples one or more structural features 14 for the structure data sampled by the first sampler 119 in descending order of the maximum energy gradient 17 (Step S36).

Since the process of Steps S37-S40 is the same as the process of Steps S17-S20 in FIG. 12. So, repetitious description is omitted here.

Effect of Second Embodiment

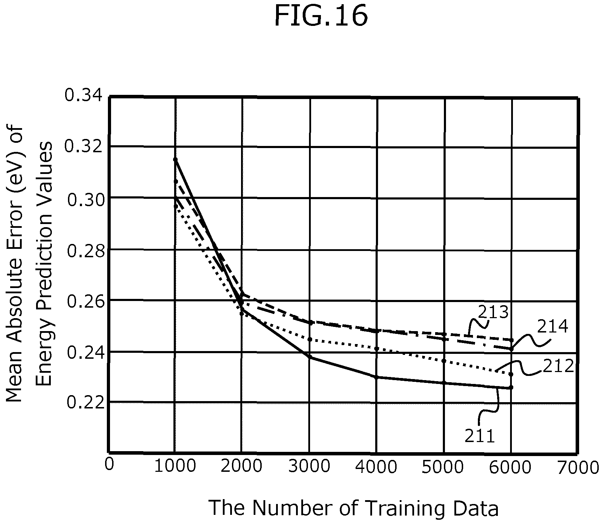

FIG. 16 is a diagram illustrating a result illustrating an effect in reducing a mean absolute error of energy prediction values of the active learning in the second embodiment. FIG. 16 illustrates an example illustrates an example in which 1000 (pieces of) training data are determined though the active learning and the energy of catalysts for generation of ammonia is predicted with the determined training data.

In FIG. 16, a polyline 211 represents a result of training using the training data determined in the active learning of the second embodiment, and a polyline 212 represents a result of training using the training data determined in the active learning of the comparative example illustrated in FIG. 3. A polyline 213 indicates the result when data is selected randomly. A polyline 214 indicates the result when the training data are selected based only on the gradients.

By training using the training data determined in the active learning of the second embodiment, the mean absolute error in the energy prediction NN 30 can be reduced as compared with training using the training data determined in the active learning of the comparative example.

In order to evaluate the active learning of the present embodiment, three catalytic datasets of OC22, catalyst for nitrogen reduction reaction, and catalyst for oxygen reduction reaction are applied to the energy prediction NN. As the energy prediction GNN, PaiNN and EquiformerV2 were used. The method of the active learning described in this embodiment have improved the accuracy of prediction under all conditions despite a small amount of the training data.

According to the active training technique of the present example, the second feature is the gradient serving as the change in the energy prediction value of the material comprising the structure for a distances of a point representing the first feature from points representing each of the multiple first features except for the point in a feature space.

This can consider the feature of the energy as well as the feature related to the structure of each material. Since the diversity of energy can be considered, structure data similar in structure but largely differing in energy can be determined to be the training data. Accordingly, by training with the determined training data, the prediction error of the energy prediction NN can be improved even if the amount of the training data is small.

In the process of determining the training data, the processor 100a calculates the maximum gradient among the gradients for each structural feature 14 in the feature space. The processor 100a samples one or more structural features 14 in descending order of the maximum gradient of each structural feature 14, and determines one or more or more structure data associated with the sampled first features to be the training data.

Since this can consider a data group having a large gradient which more highly affects the prediction error than the remaining data groups, the prediction error of the energy prediction NN can be improved even if the amount of training data is small.

As described the above, the schemes of the active learning of the first and second embodiments consider the energy prediction values as well as the feature of the material structures. Therefore, the training dataset can be sampled considering the diversities both in structure and energy of the material data. This contributes to enhancement in the accuracy of the energy prediction values, using a small amount of the training data.

REFERENCE SIGNS LIST

    1: labeled training data
    2: structure data
    3: label
    4: energy prediction value
    10: multiple structure data
    11: sampled structure data
    12: remaining structure data
    13: labeled training data
    14: structural feature
    15: energy prediction value
    16: expanded feature
    17: energy gradient
    20: DFT calculator
    30: energy prediction NN
    40: active learning NN
    41: structural feature extractor
    42: header
    100a: processor
    100b: memory
    100c: storing device
    100d: IF device
    100e: IO device
    100f: reader
    100h: recording medium
    100i: bus
    100g: program
    110: controller
    111: structure data inputting device
    112: structural feature extractor
    113: energy prediction value obtainer
    114: feature concatenator
    115: distance calculator
    116: sampler
    117: energy gradient calculator
    118: structural feature distance calculator
    119: first sampler
    120: memory unit
    132: second sampler
    121: set information

**Claims**

1. An active learning program for causing a computer to execute a process comprising:

extracting a first feature related to a structure of each of a plurality of materials by inputting a plurality of structure data (10) associated one with each of the plurality of materials into an active learning neural network (40); obtaining a second feature related to energy of the structure of each of the plurality of the materials, using the active learning neural network (40), the second feature being based on the first feature; and determining, based on the first feature and the second feature of each of the plurality of materials, one or more structure data to be training data (2) for training an energy prediction neural network (30) for predicting energy of a material from among the plurality of structure data.

2. The active learning program according to claim 1, wherein the determining comprises:

generating a concatenated feature by concatenating the first feature of each of the plurality of materials with the second feature of the material; calculating a minimum distance for each concatenated feature among distances from a point representing the concatenated feature to points representing a plurality of concatenated features in a feature space; sampling one or more concatenated features in descending order of the minimum distance for each concatenated feature; and determining one or more structure data associated with the sampled concatenated features to be the training data (2).

3. The active learning program according to claim 1 or claim 2, wherein the second feature is an energy prediction value of the material comprising the structure and is computed by the active learning neural network (40).

4. The active learning program according to claim 1 or claim 2, wherein the second feature is a gradient serving as a change in an energy prediction value of the material comprising the structure for a distance of a point representing the first feature from a point representing each of a plurality of first features except for the point in a feature space.

5. The active learning program according to claim 4, wherein the determining comprises:

calculating a maximum gradient among a plurality of the gradient of each of the first features in the feature space; sampling one or more first features in descending order of the maximum gradient of each of the first features; and

determining one or more structure data associated with the sampled first features to be the training data (2).

6. The active learning program according to any one of claims 1-5, wherein the process further comprises calculating a value of energy of the material in the structure data to be a label of the training data (2) by performing density functional theory calculation based on the structure data determined to be the training data (2).

7. A computer-implemented method for active learning comprising:

extracting a first feature related to a structure of each of a plurality of materials by inputting a plurality of structure data (10) associated one with each of the plurality of materials into an active learning neural network (40); obtaining a second feature related to energy of the structure of each of the plurality of the materials, using the active learning neural network (40), the second feature being based on the first feature; and determining, based on the first feature and the second feature of each of the plurality of materials, one or more structure data to be training data (2) for training an energy prediction neural network (30) for predicting energy of a material from among the plurality of structure data.

8. The computer-implemented method according to claim 7, wherein the determining comprises:

generating a concatenated feature by concatenating the first feature of each of the plurality of materials with the second feature of the material; calculating a minimum distance for each concatenated feature among distances from a point representing the concatenated feature to points representing a plurality of concatenated features in a feature space; sampling one or more concatenated features in descending order of the minimum distance for each concatenated feature; and determining one or more structure data associated with the sampled concatenated features to be the training data (2).

9. The computer-implemented method according to claim 7 or claim 8, wherein the second feature is an energy prediction value of the material comprising the structure and is calculated by the active learning neural network (40).

10. The computer-implemented method according to claim 7 or claim 8, wherein the second feature is a gradient serving as a change in an energy prediction value of the material comprising the structure for a distance of a point representing the first feature from a point representing each of a plurality of first features except for the point in a feature space.

11. The computer-implemented method according to claim 10, wherein the determining comprises:

calculating a maximum gradient among a plurality of the gradient of each of the first features in the feature space;
sampling one or more first features in descending order of the maximum gradient of each of the first features; and
determining one or more or more structure data associated with the sampled first features to be the training data (2).

12. The computer-implemented method according to any one of claims 7-11, further comprising calculating a value of energy of the material in the structure data to be a label of the training data (2) by performing density functional theory calculation based on the structure data determined to be the training data (2).

13. An information processing apparatus comprising a processor (100a) being configured to

extract a first feature related to a structure of each of a plurality of materials by inputting a plurality of structure data (10) associated one with each of the plurality of materials into an active learning neural network (40);
obtain a second feature related to energy of the structure of each of the plurality of the materials, using the active learning neural network (40), the second feature being based on the first feature; and
determine, based on the first feature and the second feature of each of the plurality of materials, one or more structure data to be training data (2) for training an energy prediction neural network (30) for predicting energy of a material from among the plurality of structure data.

14. The information processing apparatus according to claim 13, wherein the processor determines the training data (2) by

generating a concatenated feature by concatenating the first feature of each of the plurality of materials with the second feature of the material;
calculating a minimum distance for each concatenated feature among distances from a point representing the concatenated feature to points representing a plurality of concatenated features in a feature space;
sampling one or more concatenated features in descending order of the minimum distance for each concatenated feature; and
determining one or more structure data associated with the sampled concatenated features to be the training data (2).

15. The information processing apparatus according to claim 13 or claim 14, wherein the second feature is an energy prediction value of the material comprising the structure and is calculated by the active learning neural network (40).

# FIG.1

Supervised Learning of Neural Network

## FIG.2

Method for Training NN Applied with Active Learning

A1 — (1) Prepare Large Amount of Structure Data with Unknown Energy

A2 — (2) Input Structure Data into Active Learning NN and Sample Structure Data

A3 — (3) Calculate Energy of Sampled Structure Data by DFT and Create Labeled Training Data for Energy Prediction NN

Unlabeled Structure data

10

A4 — (4) Use Remaining Structure Data in Next Sampling for Training Data

40 — Active Learning NN

11 — Sampled Structure Data

12 — Remaining Data

13 — DFT Calculator — 20

Training — Energy Prediction NN — 30

Labeled Training Data (Structure Data After Energy Calculation

# FIG.3

**Active Learning Algorithm**

B1
Input Structure Data into ML Model and Extract Feature of Each Structure Data (Create Feature Map)

B2
Calculate Euclidean Distances between Extracted Features

B3
Sample Predetermined Number (e.g. 1,000) of Features (Structure Data) in Order of Longest Minimum Distance

**Gaussian Distributed Population**

Structure Feature SC2 (y-axis: 3, 2, 1, 0, -1, -2, -3)
Structure Feature SC1 (x-axis: -3, -2, -1, 0, 1, 2, 3)
10

$p_{1,2}$ ($p1\_min\cdots$Minimum Distance $p1\_min$ in S1)

$p_{1,3}$

S1   S2   Sn   Sn-1

P3

Structure Feature SC2 (y-axis)
Structure Feature SC1 (x-axis)

# FIG.4

Uniform Population

# FIG.5

Five-Cluster Population 10

Cluster Consisting of Single Data

Structure Feature SC2

Structure Feature SC1

# FIG.6

Energy

Si
Sj
First Data Group

Second Data Group

Structure Feature SC1/SC2

⊘ :Data Obtained in Comparative Example

◯ :Data Not Obtained in Comparative Example

# FIG.7

Data Set : O C 2 2

FIG.8

Mean Absolute Error of Energy Prediction Values of Various Data Region in Comparative Example

# FIG.9

FIG.10

# FIG.11

**100**

**Computer (Information Processing Apparatus)**

**10**
Multiple Structure Data

**110**

**111** Structure Data Inputting Device

**14** Structural Feature

**112** Structural Feature Extractor

**15** Energy Prediction Value

**113** Energy Prediction Value Obtainer

**114** Feature Concatenator

**16** Expanded Feature

**115** Distance Calculator

**116** Sampler

**120** Memory Unit

**40** Active Learning NN

**41** Structural Feature Extractor

**42** Header

**12** Remaining Structure Data

**11** Sampled Structure Data

**20** DFT Calculator

**1** Labeled Training Data

Training →

**30** Energy Prediction NN

# FIG.12

```
        ┌─────────┐
        │  Start  │
        └─────────┘
             │
             ▼
┌──────────────────────────────┐
│   Obtain Structure Data      │ ～ S10
│   with Unknown Energy        │
└──────────────────────────────┘
             │
             ▼
┌──────────────────────────────┐
│ Input Structure Data into NN │ ～ S11
│  and Extract Feature of      │ ◄──────────┐
│  Structure Data from NN      │            │
│ (Create Feature Map of       │            │
│  Structure Data)             │            │
└──────────────────────────────┘            │
             │                              │
             ▼                              │
┌──────────────────────────────┐            │
│ Predict Energy of Structure  │ ～ S12     │
│            Data              │            │
└──────────────────────────────┘            │
             │                              │
             ▼                              │
┌──────────────────────────────┐            │
│ Normalize Feature of         │ ～ S13     │
│ Structure Data and Energy    │            │
│ Prediction Value             │            │
└──────────────────────────────┘            │
             │                              │
             ▼                              │
┌──────────────────────────────┐            │
│ Concatenate Normalized       │ ～ S14     │
│ Structure Data and           │            │
│ Normalized Energy            │            │
│ Prediction Value             │            │
└──────────────────────────────┘            │
             │                              │
             ▼                              │
┌──────────────────────────────┐            │
│ Calculate Euclidean Distance │ ～ S15     │
│ between Features Using        │            │
│ Expanded Feature             │            │
└──────────────────────────────┘            │
             │              S16             │        S20
             ▼                              │
┌──────────────────────────────┐   ┌──────────────────┐
│ Sample Predetermined Number  │   │ Obtain Remaining │
│ of Features (Structure Data) │   │ Structure Data   │
│ in Descending Order of       │   └──────────────────┘
│ Distance                     │            ▲
└──────────────────────────────┘            │
             │                              │
             ▼                              │
┌──────────────────────────────┐            │
│ Create Training Data by      │ ～ S17     │
│ Labeling Sampled Structure   │            │
│ Data                         │            │
└──────────────────────────────┘            │
             │                              │
             ▼                              │
┌──────────────────────────────┐            │
│         Train NN             │ ～ S18     │
└──────────────────────────────┘            │
             │                              │
             ▼          S19                 │
           ╱────────────────────╲      No   │
          ╱  Accuracy of          ╲─────────┘
          ╲  NN Satisfies Target  ╱
           ╲  Value?             ╱
            ╲──────────────────╱
                  │ Yes
                  ▼
              ┌─────────┐
              │   End   │
              └─────────┘
```

# FIG.13

# FIG.14

**100**

**Computer (Information Processing Apparatus)**

**120**

**10** — Multiple Structure Data

**110**

**Memory Unit**

**111** — Structure Data Inputting Device

**40** — Active Learning NN

**14** — Structural Feature

**112** — Structural Feature Extractor

**41** — Structural Feature Extractor

**15** — Energy Prediction Value

**42** — Header

**113** — Energy Prediction Value Calcultor

**117** — Energy Gradient Calculator

**17** — Energy Gradient

**118** — Structural Feature Distance Calculator

**132** — Second Sampler

**12** — Remaining Structure Data

**119** — First Sampler

**11** — Sampled Structure Data

**20** — DFT Calculator

**1** — Labeled Training Data

Training

**30** — Energy Prediction NN

# FIG.15

```
        ┌─────────────┐
        │    Start     │
        └──────┬───────┘
               │
               ▼
┌──────────────────────────────┐
│   Obtain Structure Data       │ ∿ S30
│   with Unknown Energy         │
└──────────────┬───────────────┘
               │
               ▼
┌──────────────────────────────┐
│ Input Structure Data into NN  │ ∿ S31
│ and Extract                   │◄──────┐
│ Feature of Structure Data     │       │
│ from NN                       │       │
│ (Create Feature Map of        │       │
│  Structure Data)              │       │
└──────────────┬───────────────┘       │
               │                        │
               ▼                        │
┌──────────────────────────────┐       │
│ Predict Energy of Structure   │ ∿ S32 │
│ Data with NN                  │       │
└──────────────┬───────────────┘       │
               │                        │
               ▼                        │
┌──────────────────────────────┐       │
│ Calculate Euclidean Distance  │ ∿ S33 │
│ between Features Using Feature │       │
│ of Structure Data             │       │
└──────────────┬───────────────┘       │
               │                        │
               ▼                        │
┌──────────────────────────────┐       │
│ Calculate Gradients between   │ ∿ S34 │
│ Features                      │       │
└──────────────┬───────────────┘       │
               │                        │
               ▼                        │
┌──────────────────────────────┐       │
│ Sample Predetermined Number   │ ∿ S35 │
│ of Features (Structure Data)  │       │
│ in Descending Order of        │       │
│ Distance                      │       │
└──────────────┬───────────────┘       │
               │              S36       │    S40
               ▼                        │
┌──────────────────────────────┐  ┌─────────────────┐
│ Sample Predetermined Number   │  │ Obtain Remaining │
│ of Features (Structure Data)  │  │ Structure Data   │
│ in Descending Order of        │  └────────▲─────────┘
│ Gradient                      │           │
└──────────────┬───────────────┘           │
               │                            │
               ▼                            │
┌──────────────────────────────┐           │
│ Create Training Data by       │ ∿ S37     │
│ Labeling Sampled Structure    │           │
│ Data                          │           │
└──────────────┬───────────────┘           │
               │                            │
               ▼                            │
┌──────────────────────────────┐           │
│          Train NN             │ ∿ S38     │
└──────────────┬───────────────┘           │
               │            S39             │
               ▼                            │
        ◇───────────────◇                   │
       ╱  Accuracy of    ╲   No             │
      ◇ NN Satisfies      ◇──────────────────┘
       ╲ Target Value?   ╱
        ◇───────┬───────◇
                │ Yes
                ▼
        ┌─────────────┐
        │     End      │
        └─────────────┘
```

# FIG.16

# EUROPEAN SEARCH REPORT

**Application Number**

EP 25 17 9228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANONYMOUS HOJE ET AL: "Active learning accelerated exploration of single-atom local environments in multimetallic systems for oxygen electrocatalysis \| Theoretical and Computational Chemistry \| ChemRxiv \| Cambridge Open Engage", MESOSCALE AND NANOSCALE PHYSICS, vol. 10, no. 1, 14 March 2024 (2024-03-14), XP093337301, ISSN: 2057-3960, DOI: 10.1038/s41524-024-01432-1 | 1-3,6-9, 12-15 | INV. G06N3/042 G06N3/091 |
| A | * page 1 - page 12 * <br> * page 22 - page 25 * <br> ----- | 4,5,10, 11 | |
| X,P | SAKAI YASUFUMI ET AL: "Active Learning for Graph Neural Networks Training in Catalyst Energy Prediction", 2024 INTERNATIONAL JOINT CONFERENCE ON NEURAL NETWORKS (IJCNN), IEEE, 30 June 2024 (2024-06-30), pages 1-8, XP034690803, DOI: 10.1109/IJCNN60899.2024.10650978 [retrieved on 2024-09-09] * the whole document * <br> ----- | 1-15 | |
| A | SAKAI YASUFUMI ET AL: "Self-Supervised Learning with Atom Replacement for Catalyst Energy Prediction by Graph Neural Networks", PROCEDIA COMPUTER SCIENCE, ELSEVIER, AMSTERDAM, NL, vol. 222, 1 January 2023 (2023-01-01), pages 458-467, XP087387661, ISSN: 1877-0509, DOI: 10.1016/J.PROCS.2023.08.184 [retrieved on 2023-08-31] * the whole document * <br> ----- | 1-15 | |

**TECHNICAL FIELDS SEARCHED (IPC)**

G16C
G06N

The present search report has been drawn up for all claims

| Place of search | Date of completion of the search | Examiner |
|---|---|---|
| The Hague | 19 November 2025 | Jacobs, Jan-Pieter |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K. SHIMIZU et al.** Phase stability of Au-Li binary systems studied using neural network potential. *Phys. Rev. B*, 2021, vol. 103, 094112
- **K. T. SCHUETT** ; **O. T. UNKE** ; **M. GASTEGGER**. Equivariant message passing for the prediction of tensorial properties and molecular spectra. *Proceedings of the International Conference on Machine Learning*, 2021, 9377-9388

- **Y.-L. LIAO** ; **B. WOOD** ; **A. DAS** ; **T. SMIDT**. EquiformerV2: Improved equivariant transformer for scaling to higher-degree representations. *arXiv preprint arXiv:2306.12059*, 2023